# EUROPÄISCHE PATENTSCHRIFT

(11) **EP 0 543 283 B1**
(45) Veröffentlichungstag und Bekanntmachung des Hinweises auf die Patenterteilung: **27.01.1999**
(21) Anmeldenummer: 92119398.3
(22) Anmeldetag: 13.11.1992
(51) Int. Cl.: A61M 1/14

(54) **Vorrichtung zur Desinfektion von Hämodialysegeräten**
Disinfection device for hemodialysis apparatus
Dispositif de désinfection d'appareil de dialyse

(30) Priorität: 20.11.1991 DE 4138140
(43) Veröffentlichungstag der Anmeldung: 26.05.1993
(73) Patentinhaber: Fresenius Medical Care Deutschland GmbH, 61350 Bad Homburg v.d.H. (DE)
(72) Erfinder: Polaschegg, Hans-Dietrich, Dr., W-6370 Oberursel 4 (DE)
(74) Vertreter: Luderschmidt, Schüler & Partner GbR

(56) Entgegenhaltungen:
- EP-A- 0 278 100
- EP-A- 0 458 041
- US-A- 3 474 907
- US-A- 4 153 554

## Beschreibung

Die Erfindung betrifft eine Vorrichtung zur Dialyse sowie die Verwendung einer Desinfektionsvorrichtung zum Desinfizieren eines Dialysegerätes.

Vorrichtungen zur Dialyse von Blut, sogenannte Hämodialysegeräte, weisen unter anderem einen Dialysierflüssigkeits-Kreislauf auf. Dieser Kreislauf weist eine Wasserquelle und einen Dialysator auf. Wasserquelle und Dialysator sind durch eine erste Flüssigkeitsleitung miteinander verbunden. Vom Dialysator geht eine zweite Flüssigkeitsleitung ab, die vom Dialysator zum Abfluß führt. Die erste und die zweite Flüssigkeitsleitung weisen je einen Konnektor auf, der die jeweilige Leitung mit dem Dialysator verbindet. Während der Desinfektion des Dialysierflüssigkeits-Kreislaufs ist eine Desinfektionsvorrichtung in diesen Kreislauf eingeschaltet und die beiden Flüssigkeitsleitungen werden zwischen ihren Konnektoren durch ein Kurzschlußstück kurzgeschlossen.

Die Desinfektion von Hämodialyse-Geräten ist ein unerläßlicher Reinigungsschritt zwischen aufeinanderfolgenden Dialysevorgängen. Ziel der Desinfektion ist die vollständige Reinigung und Entkeimung sämtlicher Flächen und Vorrichtungen, die mit der Dialysierflüssigkeit und/oder deren Konzentraten bzw. Verdünnungsflüssigkeiten in Kontakt kommen. Eine unzureichende Desinfektion des Dialysierflüssigkeits-Kreislaufs kann zu erheblichen gesundheitlichen Schäden des behandelten Patienten führen. Der Desinfektion unterzogen werden jedoch nur die Teile des Hämodialysegerätes, die dem Dialysierflüssigkeits-Kreislauf zuzuordnen sind, da sämtliche Teile des Blutkreislaufs nach jedem Dialysiervorgang ausgetauscht und durch neue sterile Teile ersetzt werden.

Bekannt ist die Reinigung der Hämodialysegeräte, bei dem an Stelle von Dialysekonzentrat ein Desinfektionsmittelkonzentrat aus einem Kanister mit typisch 101 Konzentrat durch das Hämodialysegerät gepumpt wird, wobei die beiden Flüssigkeitsleitungen (Wasserzulauf, Dialysierflüssigkeitsablauf) nicht kurzgeschlossen werden. Nach Beenden der Desinfektion verbleibt die Desinfektionsflüssigkeit im Gerät. Vor Beginn des nächsten Dialysiervorgangs wird das Dialysegerät mit Wasser freigespült. Dieses Verfahren erfordert einen hohen Einsatz von Desinfektionsmitteln und es wird die Reinigung der Dialysiervorrichtung erreicht, die stromab der Zumischung des Desinfektionsmittels angeordnet ist. Typische Ausführungsformen dieser Vorrichtungen sind die Hämodialysegeräte A2008C-E der Anmelderin.

Verbesserte Ausführungsformen, wie sie beispielsweise in der DE 3 447 989 und in der DE 3 941 103 dargestellt sind, rezirkulieren die Desinfektionslösung nach Schaltung eines Kurzschlusses zwischen der ersten und der zweiten Flüssigkeitsleitung. Dies führt zum einen zu einer vollständigen Reinigung des Dialysierflüssigkeits-Kreislaufs und zum anderen zu einem reduzierten Verbrauch an Desinfektionsmitteln.

Als Desinfektionsmittel werden üblicherweise flüssige Reinigungsmittel, beispielsweise Formaldehyd, Peressigsäure oder Natriumhypochlorit oder ähnliche Chemikalien eingesetzt. Diese Reinigungsmittel sind aggressiv, umwelt- und gesundheitsschädlich, teilweise sogar giftig. Insbesondere Mischungen der oben genannten Reinigungsmittel erfordern größte Sorgfalt in der Anwendung, da es zur Exposion und auch zur Freisetzung elementaren Chlors kommen kann. Erst in neuerer Zeit wurde erkannt, daß Zitronensäure bei erhöhter Temperatur desinfizierend wirkt. Zitronensäure in konzentrierter Form ist ebenfalls agressiv und kann in Mischungen mit anderen Chemikalien gefährliche Reaktionen auslösen.

Abgesehen davon, daß die Transportkosten für das überwiegend aus Wasser bestehende Desinfektionsmittel hoch sind, muß eine hohe Sorgfalt beim Wechseln der Konzentrate für Dialysierflüssigkeit und Desinfektionsmittel an den Tag gelegt werden. Insofern sind besondere Vorkehrungen, beispielsweise spezielle Anschlüsse der verschiedenen Kanister an das Hämodialysegerät oder eigene Schaltungen der Flüssigkeitsleitungen, zur Vermeidung von Fehlleitungen der verschiedenen Flüssigkeiten, getroffen worden, um eine Verwechslung dieser Konzentrate zu verhindern.

Der Erfindung liegt die Aufgabe zugrunde, eine Vorrichtung zur Dialyse zu schaffen, die über eine Desinfektionsvorrichtung verfügt, mit der eine sichere und vollständige Desinfektion des Hämodialysegerätes möglich ist, ohne daß die vorstehend beschriebene Verwechslungsgefahr besteht.

Die Lösung dieser Aufgabe erfolgt mit den Merkmalen des Patentanspruchs 1.

Der Patentanspruch 11 betrifft die Verwendung einer Desinfektionsvorrichtung zum Desinfizieren eines Dialysegerätes.

Der Behälter der Desinfektionsvorrichtung kann entweder eine maschinenfeste Vorrichtung mit Verschluß oder auch ein Einmalartikel bzw. ein recyclingfähiger Behälter sein, der extern wieder aufgefüllt werden kann.

In einer vorteilhaften Ausführungsform der Erfindung sind die Anschlußstutzen gemäß DIN 58 352 ausgebildet, um Verwechslungen beim Anschließen zu vermeiden, und um universell einsetzbar zu sein.

Es ist weiter vorteilhaft, den Behälter mit einer verschließbaren Füllöffnung zu versehen, die zum Aufnehmen einer Dosismenge des nachzufüllenden Desinfektionsmittels geeignet ist.

In einer bevorzugten Ausführungsform der erfindungsgemäßen Vorrichtung ist im Innenraum des Behälters für das Desinfektionsmittel, benachbart zum Ausgangsstutzen hin, ein Filter vorgesehen, dessen Filterporen oder -gitter kleiner sind als die Pulverkorngröße des Desinfektionsmittels, wodurch pulverförmiges Desinfektionsmittel, das noch nicht gelöst ist, zurückgehalten wird.

Eine Durchströmung des Behälters der Desinfektionsvorrichtung von unten nach oben und eine tangentiale Einströmung des Wassers in den Behälter erweist sich als besonders vorteilhaft für die Verteilung und gegebenenfalls für die Auflösung des Desinfektionsmittels.

Es ist vorteilhaft, im Bereich des Ausgangsstutzens in dem nachfüllbaren Behälter ein Rückschlagventil vorzusehen, sofern ein flüssiges Desinfektionsmittel eingesetzt wird. Das Rückschlagventil kann durch den Druck des zufließenden Wassers geöffnet werden, wobei es im Betriebszustand unten angeordnet ist.

In einer besonders bevorzugten Ausführungsform der Erfindung wird zur Desinfektion pulverförmiges Desinfektionsmittel, beispielsweise kristalline oder granulierte Zitronensäure, verwendet. Die Verwendung fester Desinfektionsmittel hat den besonderen Vorteil, daß bis dahin gelegentlich vorkommende Verwechslungen von Desinfektions- und Dialysierflüssigkeit ausgeschlossen sind. Damit wird die Desinfektion der Hämodialysegeräte erheblich sicherer gestaltet. Die Zitronensäure wird dabei in geeigneter Weise dosiert, beispielsweise in einer Menge von 5 - 30 g/l des zu desinfizierenden Volumens, wobei der Behälter eine solche Menge aufnehmen und im Füllzustand ein Durchströmen des Behältes ohne Behinderung stattfinden kann. Somit wird sichergestellt, daß eine ausreichend hoch konzentrierte Zitronensäurelösung im Hämodialysegerät rezirkuliert wird, und eine sichere und vollständige Desinfektion gewährleistet ist.

In einer anderen Ausführungsform der Erfindung kann auch flüssiges Reinigungsmittel, beispielsweise Peressig-, Formaldehyd- oder Natriumhypochloritlösungen, in den verschließbaren Behälter der Desinfektionsvorrichtung nachgefüllt werden. Hier bietet die Verwendung abgepackter Konzentratlösungen ebenfalls den erheblichen Vorteil, daß sich die Nachfüllpackungen unverwechselbar von den Behältern der Dialysierflüssigkeit unterscheiden, so daß Verwechslungen besser als bisher vermieden werden können. Zudem bietet die Verwendung von Reinigungsmittel-Konzentraten deutliche Vorteile insofern, als das Transportvolumen des Reinigungsmittels deutlich reduziert wird und damit Kostenvorteile erzielt werden.

Während der Dialyse sind die Dialysierflüssigkeits-Schläuche mit dem Dialysator verbunden. Nach Abschluß der Dialyse wird der Dialysator entfernt, und die entsprechenden Konnektoren der ersten und zweiten Flüssigkeitsleitung werden mit dem Behälter der Desinfektionseinrichtung verbunden. Vorzugsweise ist am Hämodialysegerät eine Halterung mit einem Sensor angeordnet, mit dem die Fixierung des Behälters sicher festgestellt werden kann, so daß die Existenz und die zutreffende Anordnung der Desinfektionseinrichtung jederzeit einfach und sicher überwacht werden kann.

Um weiterhin die sichere Überwachung der Desinfektionseinrichtung zu gewährleisten, sind in einer bevorzugten Ausführungsform der Erfindung in den Konnektoren, die den nachfüllbaren Behälter der Desinfektionsvorrichtung mit den ersten und zweiten Flüssigkeitsleitungen verbinden, weitere Sensoren vorgesehen, mittels derer die sichere Verbindung der Konnektoren mit dem Behälter überprüfbar ist, und die erst bei Bestehen dieser Verbindung den Beginn des Desinfektionsbetriebs zulassen.

Um eindeutig zwischen einem einfachen Spülvorgang des Hämodialysegerätes und einer vollständigen Desinfektion unterscheiden zu können, kann der Behälter der Desinfektionsvorrichtung über ein elektrisch ansteuerbares oder mechanisch betätigbares und elektrisch lesbares Bypassventil angesteuert werden.

In einer vorteilhaften Ausführungsform der vorliegenden Erfindung sind Zuführleitung, Abführleitung und/oder Dialysierflüssigkeitsquelle über eine Rezirkulationsleitung verbunden und die Vorrichtung weist eine Absperreinrichtung zum Herstellen des Rezirkulationskreislauf auf. Mit dem derart geschlossenem Rezirkulationskreislauf wird die vollständige Reinigung aller Teile bzw. Oberflächen des Dialysierflüssigkeits-Kreislaufs sichergestellt.

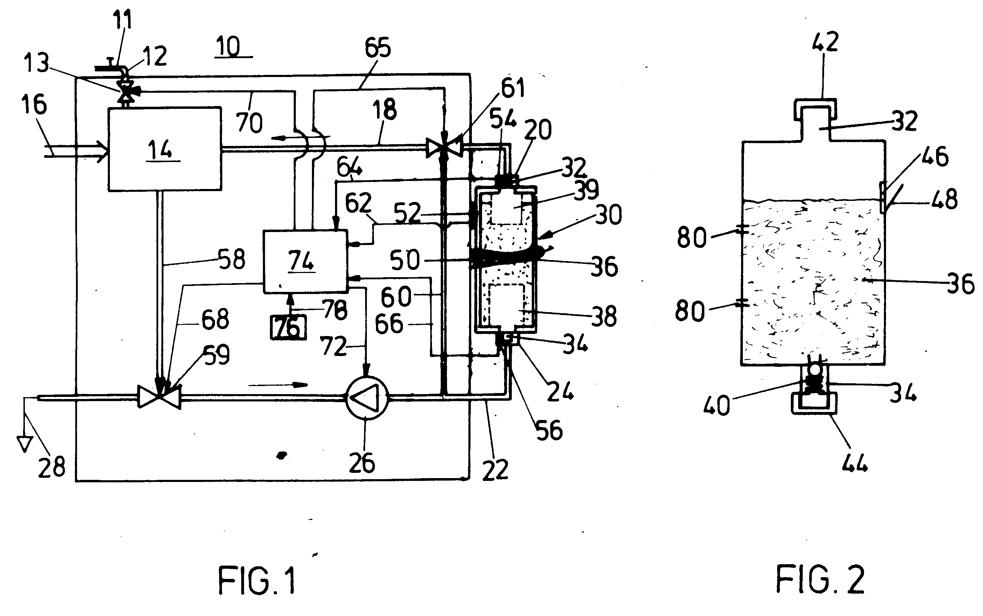

Eine bevorzugte Ausführungsform der Erfindung ist nachfolgend in den Fig. 1 und 2 dargestellt:
- Fig. 1: Schema eines Hämodialysegeräts während des Desinfektionsvorgangs in Schnittansicht
- Fig. 2: Schnitt durch einen Behälter der Desinfektionsvorrichtung

Das in Figur 1 dargestellte Hämodialysegerät 10 weist eine Wasserquelle 11 auf, die über eine Leitung 12, in die ein Ventil 13 eingeschaltet ist, in einen Mischbehälter 14 mündet, der wiederum mit einer Zuführung 16 für ein Elektrolytkonzentrat verbunden ist. Von dem Mischbehälter 14 geht eine erste Flüssigkeitsleitung 18 ab, die an ihrem Ende aus dem Hämodialysegerät 10 herausragt und einen ersten Konnektor 20 aufweist. Dieser erste Konnektor 20 ist üblicherweise während des Dialysebetriebs mit einem nicht gezeigten Dialysator verbunden.

In das Hämodialysegerät 10 ist eine zweite Flüssigkeitsleitung 22 zurückgeführt, die an ihrem außerhalb des Hämodialysegeräts 10 befindlichen Endes einen zweiten Konnektor 24 aufweist, der im Dialysebetrieb mit dem Ausgang des nicht dargestellten Dialysators verbunden ist.

Innerhalb des Hämodialysegeräts 10 ist in die zweite Leitung 22 eine Pumpe 26 eingeschaltet. Das andere Ende der zweiten Leitung 22 ist schließlich mit dem Abfluß 28 verbunden. Ein solches Dialysegerät ist Stand der Technik und somit nicht Gegenstand der Erfindung.

In Figur 1 ist das Hämodialysegerät 10 während der Desinfektionsphase dargestellt. Hierzu ist zwischen den beiden Konnektoren 20 und 24 ein Desinfektionsmittelbehälter 30 vorgesehen, der vorteilhafterweise eine hohlzylindrische Form aufweist. Dieser Desinfektionsmittelbehälter 30 weist an seinen beiden Enden jeweils einen Zuführungsstutzen 32 und 34 auf. Diese beiden Stutzen 32 und 34 können formschlüssig mit den beiden Konnektoren 20 und 24 verbunden werden und sind, da die Stutzen des nicht gezeigten Dialysators international genormt sind, vorteilhafterweise mit der Form dieser Dialysatorstutzen identisch. Sie weisen gemäß dieser Ausführungsform den Anschluß gemäß DIN 58 352 auf. Im übrigen sind die Konnektoren 20 und 24 entsprechend dieser Norm ausgebildet. Gemäß einer bevorzugten Ausführungsform ist zumindest der Stutzen 32 oder 34, durch den die Flüssigkeit in den Behälter 30 einströmt, tangential zur Längsachse des hohlzylidrisch ausgebildeten Behälters 30 angeordnet, um das Aus- und Einströmen der Flüssigkeit in den Behälter 30 zu optimieren.

Der Desinfektionsmittelbehälter 30 ist mit einem Desinfektionsmittel 36 gefüllt, das, wie in Figur 1 dargestellt ist, eine körnige, d.h. pulverförmige Struktur aufweisen kann.

Im Bereich des Ausgangsstutzens 34 ist innerhalb des Desinfektionsmittelbehälters 30 ein gitterförmiges zylindrisches Filter 38 vorgesehen, das allseits geschlossen ist und eine mittlere Maschenweite aufweist, die kleiner ist als die Partikel des Desinfektionsmittels. Dieses Filter 38 hat die Aufgabe, nicht gelöstes Desinfektionsmittel, innerhalb des Desinfektionsmittelbehälters 30 zurückzuhalten. In der in Figur 2 dargestellten Ausführungsform ist innerhalb des Desinfektionsmittelbehälters 30 ein flüssiges Desinfektionsmittelkonzentrat 36 vorgesehen. Der in Figur 2 dargestellte Behälter 30 ist in seiner Gebrauchslage angeordnet, d.h. der Abflußstutzen 34 befindet sich unten. Um ein Ausfließen des flüssigen Desinfektionsmittels 36 beim Konnektieren zu verhindern, ist im Abflußstutzen 34 ein Rückschlagventil 40 vorgesehen, das durch den Druck des zufließenden Wasser-Desinfektionsmittelgemischs geöffnet werden kann.

Vor Gebrauch weist der Desinfektionsmittelbehälter 30 an seinen beiden Anschlüssen 32 und 34 jeweils Kappen 42 und 44 auf, mit denen die beiden Anschlüsse 32 und 34 verschlossen sind und die im Bedarfsfall geöffnet werden können.

Desweiteren kann in dem Behälter 30 eine Einfüllöffnung 46 vorgesehen sein, die mit einem Deckel 48 verschlossen wird.

An dem Hämodialysegerät 10 ist eine Halterung 50 für den Desinfektionsmittelbehälter 30 angeordnet, die im Beispielsfall in Form von zwei auseinanderklappbaren Haltebacken 50 dargestellt ist. Desweiteren befindet sich in der Nähe dieser Halterung 50 ein erster Sensor 52 am Hämodialysegerät 10, der beim Einlegen des Behälters 30 in die Halterung 50 durch diesen betätigt wird und eine vorbestimmte Anordnung des Behälters 30 anzeigt. Dieser Sensor 52 wird durch diesen Behälter 30, nicht jedoch durch den Dialysator aktiviert, sofern dieser in die Halterung 50 eingelegt wird. Hierdurch wird sichergestellt, daß ein Behälter 30 eingelegt und in einer vorbestimmten Anordnung am Dialysegerät 10 angeordnet ist.

Desweiteren können die Konnektoren 20 und 24 mit Kontaktsensoren 54 und 56 ausgerüstet sein, die gemäß Figur 1 symbolisch über eine Verbindungsleitung mit dem Hämodialysegerät 10 verbunden sind. Mit diesen Kontaktsensoren 54 und 56 kann die Verbindung der Konnektoren 20 und 24 mit den Anschlüssen 32 und 34 ermittelt werden.

In einer weiteren Ausführungsform ist die zweite Leitung 22 über eine Bypass-Leitung 58 mit dem Mischbehälter 14 verbunden, wobei der Verbindungspunkt zwischen der Bypass-Leitung 58 und der Leitung 22 eine Ventilanordnung 59 aufweist, mit der die zweite Leitung 22 wahlweise mit dem Ausfluß 28 oder im Rezirkulationsbetrieb über die Bypass-Leitung 58 mit dem Mischbehälter 14 verbunden werden kann.

Die Zuleitung 16 und die Ableitung 22 sind mit der üblichen Bypassleitung 60 verbunden, wobei am Verbindungspunkt der beiden Leitungen ein Bypassventil 61 vorgesehen ist. Die Sensoren 52-56, die Ventilanordnung 59, das Frischwasserventil 13 und die Dialysierflüssigkeitspumpe 26 sind über Leitungen 62-72 mit einer Steuereinheit 74 verbunden, die mit einer Eingabeeinheit 76 über die Leitung 78 verbunden ist.

Die Steuereinheit 74 zur Durchführung der Desinfektion kann - wie durch die angedeuteten Pfeile dargestellt - erst dann betätigt werden, wenn die Sensoren 52-56 das Verbindungssignal über die Leitungen 62-66 an die Steuereinheit 74 abgegeben haben und die Eingabeeinheit 76 vom Bediener aktiviert worden ist.

Das Hämodialysegerät 10 wird zur Aufnahme der Desinfektion folgendermaßen betrieben:
Zunächst wird der Dialysator von den Konnektoren 20 und 24 abgehängt. Anschließend wird der mit frischem Desinfektionsmittel gefüllte Behälter 30 an seinen Stutzen 32 und 34 mit den beiden Konnektoren 20 und 24 verbunden, wobei die Kontaktsensoren 54 und 56 eine formschlüssige Verbindung sicherstellen und der Steuereinheit 74 anzeigen.

Sodann wird der Behälter 30 in eine vorbestimmte Lage zum Hämodialysegerät 10 gebracht, was dadurch geschieht, daß er in die Halterung 50 eingelegt wird und dort den Sensor 52 betätigt, der die vorgelegte zutreffende Anordnung über die Verbindungsleitung 62 an die Steuereinheit 74 weiterliefert. Das Desinfektionsprogramm kann nun durch Betätigen der Eingabeeinheit 76 gestartet werden. Hierzu wird die Wasserquelle 11 durch Öffnen des Ventils 13 betätigt und es wird die Ventilanordnung 59 in den Rezirkulationsbetrieb geschaltet. Gemäß einem ersten vorbestimmten Programmablauf, der in der Steuereinheit 74 abgelegt ist, wird dann das Wasser durch die gesamte Leitungsanordnung 18, 22, 58, 14 geführt, und zwar so lange, bis die gesamte Desinfektionsmittelmenge einheitlich innerhalb dieses Leitungssystems verteilt ist. Anschließend läßt man die Desinfektionslösung so lange auf das Hämodialysegerät 10 einwirken, bis das gesamte Dialysesystem sicher desinfiziert ist. Danach wird mit Frischwasser dadurch gespült, daß die Ventilanordnung 59 wechselweise mit dem Abfluß 28 und der Rezirkulationsleitung 58 zum Freispülen verbunden wird. Andererseits kann man jedoch auch das Gerät 10 mit der Desinfektionslösung gefüllt lassen und erst kurz vor dem erneuten Gebrauch freispülen. Schließlich kann es vorteilhaft sein, das Bypassventil 61 über die Verbindungsleitung 65 zu betätigen, wenn der Behälter 30 nicht, jedoch die restliche Vorrichtung mit Wasser oder Desinfektionslösung durchströmt werden soll. Das nunmehr sterile, mit Frischwasser gefüllte Hämodialysegerät 10 kann danach einer weiteren Dialyse zugeführt werden. Hierzu wird der Desinfektionsmittelbehälter 30 von den Konnektoren 20 und 24 abgehängt, die mit einem sterilen, nicht gebrauchten Dialysator anschließend verbunden werden.

Gemäß einem weiteren Verfahren, dessen Parameter ebenfalls in der Steuereinheit 74 abgelegt wird, wird der Behälter 30 in der Betriebslage von unten nach oben durchströmt. Hierzu wird die Pumpe 26 von der Steuereinheit in die umgekehrte Pumprichtung umgeschaltet. Diese Verfahrensart hat den Vorteil, daß das im Behälter 30 vorliegende Pulver 36 nach oben aufgewirbelt wird, so daß sich der Abflußstutzen 34 nicht zusetzt. Andererseits kann eine solche Betriebsart auch dadurch erreicht werden, daß das Ende der Zuführungsleitung 18 mit ihrem Konnektor 20 in der Gebrauchslage unten am Behälter 30 montiert wird, während die Abflußleitung 22 oben am Behälter 30 angebracht wird. In diesem Fall ist dann die Umkehrung der Förderrichtung der Pumpe 16 nicht notwendig. Im übrigen ist es bei einer solchen Durchströmung des Behälters 30 von unten nach oben vorteilhaft, ein Filter 39, das dem Filter 38 entsprechen kann, am oberen Stutzen 32 anzuordnen, um ein Ausströmen von nicht gelöstem Pulver zu verhindern.

Wird beispielsweise festes Zitronensäuregranulat zur Desinfektion eingesetzt, so reichen zur Einstellung einer Desinfektionsmittel-Konzentration von 0,5-3 % bei einem Füllvolumen des Hämodialysegeräts von 2 l und einer Dichte der granulierten Zitronensäure von ca. 1 zwischen 10 und 60 g (entsprechend einem benötigten Volumen von weniger als 100 ml) zur Füllung des Behälters 30 aus. Eine solche Menge Desinfektionsmittel kann über die Einfüllöffnung 46 in den leeren Behälter 30 eingefüllt werden, der anschließend erneut zur Desinfektion eingesetzt werden kann. Um eine sichere Einfüllung der benötigten Desinfektionsmittelmenge zu gewährleisten, kann der Behälter 30 an seiner Außenseite Markierungen 80 aufweisen, die, sofern ein durchsichtiges Kunststoffmaterial, beispielsweise Polycarbonat, eingesetzt wird, die zur Ermittlung der Füllstandshöhe und damit der eingefüllten Menge Desinfektionsmittel eingesetzt werden können.

## Patentansprüche

1. Vorrichtung zur Dialyse (10), aufweisend eine Dialysierflüssigkeitsquelle (14) mit einer Wasserquelle (11), von der sich eine Zuführungsleitung (18) erstreckt, deren Ende über einen ersten Konnektor (20) mit einem Dialysator verbindbar ist, einer Abführleitung (22), deren eines Ende über einen zweiten Konnektor (24) mit dem Dialysator verbindbar ist, und deren anderes Ende zum Abfluß (28) führt, sowie mit einer Desinfektionsvorrichtung, die im Desinfektionsbetrieb mit der Dialysierflüssigkeitsquelle (14) und den beiden im Desinfektionsbetrieb kurzgeschlossenen Leitungen (18, 22) verbunden ist, dadurch gekennzeichnet, daß die Desinfektionsvorrichtung einen Behälter (30) mit zwei Anschlußstutzen (32, 34) zum Anschluß an den ersten Konnektor (20) und den zweiten Konnektor (24) aufweist, wobei der Behälter (30) eine vorbestimmte Dosis Desinfektionsmittel (36) aufweist.

2. Vorrichtung nach Anspruch 1, dadurch gekennzeichnet, daß die Anschlußstutzen (32, 34) als Anschluß gemäß DIN 58 352 ausgeführt sind.

3. Vorrichtung nach Anspruch 1 oder 2, dadurch gekennzeichnet, daß der Behälter (30) eine mit einem Deckel (48) verschließbare Füllöffnung (46) zum Nachfüllen einer Dosismenge Desinfektionsmittel (36) aufweist.

4. Vorrichtung nach Anspruch 1, 2 oder 3, dadurch gekennzeichnet, daß im Behälter (30) benachbart zum Ausgangsstutzen (34) hin ein Filter (38) vorgesehen ist, und der Behälter (30) pulverförmiges Desinfektionsmittel (36) aufweist.

5. Vorrichtung nach einem der vorangehenden Ansprüche, dadurch gekennzeichnet, daß im Bereich des Ausgangsstutzens (34) ein Rückschlagventil (40) vorgesehen ist, das durch den Druck des zufließenden Wassers geöffnet werden kann und ein flüssiges Desinfektionsmittel (36) im Behälter (30) vorgesehen ist.

6. Vorrichtung nach einem der vorangehenden Ansprüche, dadurch gekennzeichnet, daß an der Wand des Hämodialysegeräts (10) eine Halterung (50) und ein Sensor (52) angeordnet sind, mit dem eine behälterspezifische Fixierung des Behälters (30) in der Halterung (50) feststellbar ist.

7. Vorrichtung nach einem der vorangehenden Ansprüche, dadurch gekennzeichnet, daß Kontaktsensoren (54, 56) in den Konnektoren angeordnet sind, mit denen eine Verbindung der Konnektoren (20, 24) mit den Anschlußstutzen (32, 34) des Behälters (30) feststellbar ist.

8. Vorrichtung nach einem der vorangehenden Ansprüche, dadurch gekennzeichnet, daß Zuführleitung (18), Abführleitung (22) und der Mischbehälter (14) über eine Rezirkulationsleitung (58) verbunden sind und eine Absperreinrichtung (59) zum Herstellen des Rezirkulationskreislaufs zwischen Rezirkulationsleitung (58) und Abführleitung (22) angeordnet ist.

9. Vorrichtung nach einem der vorangehenden Ansprüche, dadurch gekennzeichnet, daß das Desinfektionsmittel (36) Zitronensäure ist und in einer Menge von 5 - 30 g/l, bezogen auf das zu desinfizierende Volumen, im Behälter (30) vorliegt.

10. Vorrichtung nach einem der Ansprüche 1 bis 8, dadurch gekennzeichnet, daß als Desinfektionsmittel (36) Peressigsäure, wäßrige Formaldehydlösung oder Natriumhypochloritlösung im Behälter (30) vorliegt.

11. Verwendung einer Desinfektionsvorrichtung zum Desinfizieren eines Dialysegerätes, das eine Dialysierflüssigkeitsquelle (14) mit einer Wasserquelle (11), von der sich eine Zuführungsleitung (18) erstreckt, deren Ende über einen ersten Konnektor (20) mit einem Dialysator verbindbar ist und eine Abführleitung (22) aufweist, deren eines Ende über einen zweiten Konnektor mit dem Dialysator verbindbar ist und deren anderes Ende zu einem Abfluß (28) führt, wobei die Desinfektionsvorrichtung einen mit einer vorbestimmten Dosis Desinfektionsmittel befüllten Behälter (30) mit einem Eingangsstutzen (32) zum Anschluß an den ersten Konnektor (20) und einen Ausgangsstutzen (34) zum Anschluß an den zweiten Konnektor (24) aufweist.

12. Ausführungsform nach Anspruch 11, dadurch gekennzeichnet, daß im Behälter (30) benachbart zum Ausgangsstutzen (34) ein Filter (38) vorgesehen ist und der Behälter mit pulverförmigem Desinfektionsmittel (36) befüllt ist.

13. Ausführungsform nach Anspruch 11, dadurch gekennzeichnet, daß im Bereich des Ausgangsstutzens (34) ein Rückschlagventil (40) vorgesehen ist, das durch den Druck des zufließenden Wassers geöffnet werden kann, und der Behälter (30) mit flüssigem Desinfektionsmittel befüllt ist.

## Claims

1. A dialysis apparatus (10) comprising a dialysing fluid source (14) with a water source (11) from which extends a feed line (18) the end of which can be connected via a first connector (20) to a dialyser, a discharge line (22), one end of which can be connected via a second connector (24) to the dialyser and of which the other end leads to the drain (28), and with a disinfection device which, in disinfection mode, communicates with the dialysing fluid source (14) and the two lines (18, 22) which are short-circuited in disinfection mode, characterised in that the disinfection device comprises a container (30) having two connecting unions (32, 34) for connection to the first connector (20) and the second connector (24), the container (30) comprises a predetermined dose of disinfectant (36).

2. An apparatus according to claim 1, characterised in that the connecting unions (32, 34) are constructed as a connection according to DIN 58 352.

3. An apparatus according to claim 1 or 2, characterised in that the container (30) comprises a filling aperture (46) adapted to be occluded by a cover (48) and which is intended for topping up a measured quantity of disinfectant (36).

4. An apparatus according to claim 1, 2 or 3, characterised in that in the container (30), adjacent to the outlet connector (34), there is a filter (38) while the container (30) comprises powdered disinfectant (36).

5. An apparatus according to one of the preceding claims, characterised in that in the area of the outlet connector (34) there is a non-return valve (40) which can be opened by the pressure of the incoming water, a liquid disinfectant (36) being present in the container (30).

6. An apparatus according to one of the preceding claims, characterised in that on the wall of the haemodialysis unit (10) there is a holder (50) and a sensor (52) with which it is possible to establish a container-specific fixing of the container (30) in the holder (50).

7. An apparatus according to one of the preceding claims, characterised in that contact sensors (54, 56) are disposed in the connectors, with which it is possible to establish a connection of the connectors (20, 24) to the connecting unions (32, 34) of the container (30).

8. An apparatus according to one of the preceding claims, characterised in that feed line (18), discharge line (22) and the mixing container (14) are connected by a recirculation line (58) and in that a shut-off means (59) for establishing the recirculation circuit is disposed between the recirculation line (58) and the discharge line (22).

9. An apparatus according to one of the preceding claims, characterised in that the disinfectant (36) is citric acid and is present in the container (30) in a quantity of 5 to 30 g/litre with reference to the volume to be disinfected.

10. An apparatus according to one of claims 1 to 8, characterised in that peracetic acid, aqueous formaldehyde solution or sodium hypochlorite solution is present in the container (30) as a disinfectant (36).

11. Use of a disinfection device for the disinfection of a dialysis unit and which comprises a dialysing fluid source (14) with a water source (11) from which extends a feed line (18) the end of which can be connected via a first connector (20) to a dialyser and a discharge line (22), one end of which can be connected to the dialyser via a second connector while its other end leads to an outlet (28), the disinfection device comprising a container (30) filled with a predetermined dose of disinfectant and which has an inlet connector (32) for connection to the first connector (20) and an outlet connector (34) for connection to the second connector (24).

12. An embodiment according to claim (11), characterised in that a filter (38) is provided in the container (30) adjacent the outlet connector (34) and in that the container is filled with powdered disinfectant (36).

13. An embodiment according to claim 11, characterised in that there is in the area of the outlet connector (34) a non-return valve (40) which can be opened by the pressure of the incoming water, and in that the container (30) is filled with liquid disinfectant.

## Revendications

1. Dispositif pour dialyse (10) présentant une source de liquide de dialyse (14) comprenant une source d'eau (11) à partir de laquelle s'étend un conduit d'amenée (18) dont l'extrémité peut être reliée via un premier connecteur (20) à un dialyseur, un conduit d'évacuation (22) dont la première extrémité peut être reliée via un deuxième connecteur (24) au dialyseur et dont l'autre extrémité mène à l'égout (28), ainsi qu'un dispositif de désinfection qui est relié, lorsque la désinfection est mise en service, à la source de liquide de dialyse (14) et aux deux conduits (18, 22) court-circuités lorsque la désinfection est mise en service, caractérisé en ce que le dispositif de désinfection présente un récipient (30) comprenant deux tubulures de raccord (32, 34) pour le raccordement au premier connecteur (20) et au second connecteur (24), le récipient (30) présentant une dose prédéterminée d'un désinfectant (36).

2. Dispositif selon la revendication 1, caractérisé en ce que les tubulures de raccord (32, 34) sont réalisées sous forme de raccords selon la norme DIN 58 352.

3. Dispositif selon la revendication 1 ou 2, caractérisé en ce que le récipient (30) présente une ouverture de remplissage (42) qui peut être fermée à l'aide d'un couvercle (48), pour la recharge d'une quantité dosée de désinfectant (36)

4. Dispositif selon la revendication 1, 2 ou 3, caractérisé en ce qu'on prévoit dans le récipient (30), un filtre (38) en position adjacente à la tubulure de sortie (34), le récipient (30) présentant un désinfectant pulvérulent (36).

5. Dispositif selon l'une quelconque des revendications précédentes, caractérisé en ce qu'on prévoit une soupape anti-retour (40) dans la zone de la tubulure de sortie (34), qui peut être ouverte sous l'influence de la pression exercée par le courant d'eau d'amenée, un désinfectant liquide (36) étant prévu dans le récipient (30).

6. Dispositif selon l'une quelconque des revendications précédentes, caractérisé en ce qu'un support (50) et un palpeur (52) sont disposés contre la paroi de l'appareil d'hémodialyse (10), palpeur avec lequel on peut déterminer une fixation du récipient (30) spécifique à ce dernier dans le support (50).

7. Dispositif selon l'une quelconque des revendications précédentes, caractérisé en ce que des palpeurs (54, 56) travaillant par contact sont disposés dans les connecteurs, avec lesquels on peut déterminer une liaison des connecteurs (20, 24) avec les tubulures de raccord (32, 34) du récipient (30).

8. Dispositif selon l'une quelconque des revendications précédentes, caractérisé en ce que le conduit d'amenée (18), le conduit d'évacuation (22) et le récipient de mélange (14) sont reliés via un conduit de recyclage (58), un dispositif de blocage (59) pour l'obtention du circuit de recyclage étant disposé entre le conduit de recyclage (58) et le conduit d'évacuation (22).

9. Dispositif selon l'une quelconque des revendications précédentes, caractérisé en ce que le désinfectant (36) est de l'acide citrique et est présent dans le récipient (30) en une quantité de 5 à 30 g/l rapportés au volume à désinfecter.

10. Dispositif selon l'une quelconque des revendications 1 à 8, caractérisé en ce que, à titre de désinfectant (36), est présent dans le récipient (30), de l'acide peracétique, une solution aqueuse de formaldéhyde ou une solution aqueuse d'hypochlorite de sodium.

11. Utilisation d'un dispositif de désinfection pour désinfecter un appareil de dialyse qui présente une source de liquide de dialyse (14) comprenant une source d'eau (11) à partir de laquelle s'étend un conduit d'amenée (18) dont l'extrémité peut être reliée via un premier connecteur (20) à un dialyseur, ainsi qu'un conduit d'évacuation (22) dont la première extrémité peut être reliée au dialyseur via un deuxième connecteur et dont l'autre extrémité mène à l'égout (28), dans lequel le dispositif de désinfection présente un récipient (30) rempli avec une dose prédéterminée de désinfectant, comprenant une tubulure d'entrée (32) pour être raccordée au premier connecteur (20) et une tubulure de sortie (34) pour être raccordée au deuxième connecteur (24).

12. Forme de réalisation selon la revendication 11, caractérisée en ce qu'on prévoit, dans le récipient (30), un filtre (38) en position adjacente à la tubulure de sortie (34), le récipient étant rempli avec un désinfectant pulvérulent.

13. Forme de réalisation selon la revendication 11, caractérisée en ce qu'on prévoit une soupape antiretour (40) dans la zone de la tubulure de sortie (34), qui peut être ouverte sous l'influence de la pression exercée par le courant d'eau d'amenée, le récipient (30) étant rempli avec un désinfectant liquide.
